# EUROPEAN PATENT APPLICATION

(11) **EP 3 017 788 A1**
(43) Date of publication of application: **11.05.2016**
(21) Application number: 14820252.6
(22) Date of filing: 12.06.2014
(51) Int. Cl.: A61C 17/20, A61C 3/03, A61C 17/00, A61C 17/02, A61L 2/18

(54) **DENTAL TREATMENT DEVICE**

(30) Priority: 03.07.2013 JP 2013139406
(71) Applicant: A-Z LTD., Sendai-shi Miyagi 980-0801 (JP)
(72) Inventor: KANNO, Taro, Sendai-shi Miyagi 980-0871 (JP); NAKAMURA, Keisuke, Sendai-shi Miyagi 980-0803 (JP); NIWANO, Yoshimi, Osakasayama-shi Osaka 589-0021 (JP); KANNO, Minoru, Sendai-shi Miyagi 980-0871 (JP)
(74) Representative: Pulieri, Gianluca Antonio
(86) International application number: PCT/JP2014/065554
(87) International publication number: WO 2015/001933

(57) **Abstract**

A dental treatment device in which attenuation of a laser beam is not liable to occur is provided. This dental treatment device (1) is provided with a vibration unit (2) for vibrating by a vibrator (21) that generates ultrasonic vibrations, a chip unit (3) connected to the vibration unit (2) and through which ultrasonic vibrations are transmitted, a flow channel (4) for discharging an antimicrobial agent from the chip unit (3), and an optical system (5) for irradiating a laser beam for photo-decomposing the antimicrobial agent from the chip unit (3), the optical system (5) having an optical path separate from the flow channel (4).

## Description

### TECHNICAL FIELD

The present invention relates to a dental treatment device used for prevention and treatment of periodontal disease in dental therapy.

### BACKGROUND ART

In recent years, an inflammatory disease caused due to propagation of causative bacteria in a periodontal pocket, i.e., so-called "periodontal disease," has been an issue. In order to treat such periodontal disease, cleaning (cleanup) of dental calculus or plaque is performed by a dental scaler using ultrasonic vibration.

Since many bacteria adhere to dental calculus or plaque, portions from which the dental calculus etc. are to be removed or have been removed are preferably disinfected in the case of removing the dental calculus or plaque. For disinfection/sterilization, e.g., an autoclave device is used. In the autoclave device, an object to be disinfected/sterilized is dipped in highpressure high-temperature vapor for several tens of minutes to perform sterilization.

A dental calculus removing and disinfecting device described in Patent Literature 1 has been known as a device capable of simultaneously performing the cleaning and the disinfection/sterilization as described above. In this dental calculus removing and disinfecting device, while dental calculus is crushed using ultrasonic vibrations, an antiseptic solution supplied from a tip end portion is irradiated with light to generate hydroxyl radicals for disinfection.

### CITATION LIST

### PATENT LITERATURE

PATENT LITERATURE 1: JP-A-2012-75602

### SUMMARY OF INVENTION

### TECHNICAL PROBLEM

However, the dental calculus removing and disinfecting device of Patent Literature 1 described above is configured such that a laser beam passes through a hydrogen peroxide solution. For this reason, there is a problem that great attenuation of the laser beam is caused due to scattering of the laser beam by the hydrogen peroxide solution.

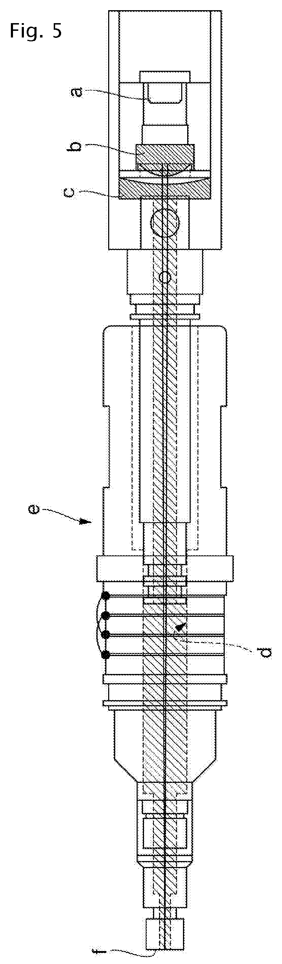

That is, in a conventional case as illustrated in Fig. 5, a laser beam generated from a light source a is condensed by condensing lenses b, c, and then, is transmitted in an antimicrobial agent contained in a flow channel d. Thus, the laser beam is scattered by the antimicrobial agent, and is greatly attenuated. Then, such a laser beam reaches a tip end f of a vibration unit e.

The present invention is intended to provide a dental treatment device in which attenuation of a laser beam is not liable to occur.

### SOLUTION TO PROBLEM

In order to accomplish the above-described goal, a dental treatment device of the present invention is a dental treatment device for removing dental calculus or plaque and disinfecting teeth, which includes a vibration unit configured to vibrate by a vibrator configured to generate ultrasonic vibrations; a chip unit which is connected to the vibration unit and to which the ultrasonic vibrations are transmitted; a flow channel configured to discharge an antimicrobial agent from the chip unit; and an optical system configured to irradiate a laser beam for photo-decomposing the antimicrobial agent from the chip unit, the optical system having an optical path separated from the flow channel.

### ADVANTAGEOUS EFFECTS OF INVENTION

As described above, the dental treatment device of the present invention is the dental treatment device for removing dental calculus or plaque and disinfecting teeth. This dental treatment device includes the vibration unit configured to vibrate by the vibrator configured to generate ultrasonic vibrations, the chip unit which is connected to the vibration unit and to which the ultrasonic vibrations are transmitted, the flow channel configured to discharge the antimicrobial agent from the chip unit, and the optical system configured to irradiate the laser beam for photo-decomposing the antimicrobial agent from the chip unit, the optical system having the optical path separated from the flow channel. With this configuration, scattering of the laser beam by the antimicrobial agent is not liable to occur. Thus, a high-intensity laser beam is irradiated from a tip end of the chip unit, leading to efficient photo-decomposition of the antimicrobial agent.

### BRIEF DESCRIPTION OF DRAWINGS

[FIG. 1] Fig. 1 is a perspective view illustrating an entire configuration of a dental treatment device.
[FIG. 2] Fig. 2 is a cross-sectional view illustrating configurations of a vibration unit and a shaft portion according to an embodiment.
[FIG. 3A] Fig. 3A is a cross-sectional view schematically illustrating the configuration of the shaft portion.
[FIG. 3B] Fig. 3B is a side view schematically illustrating the configuration of the shaft portion on a tip end side thereof.
[FIG. 4A] Fig. 4A is a cross-sectional view illustrating a configuration of spacer members.
[FIG. 4B] Fig. 4B is a perspective view illustrating the configuration of the spacer members.
[FIG. 5] Fig. 5 is a cross-sectional view illustrating configurations of a vibration unit and a shaft portion according to a conventional example.

### DESCRIPTION OF EMBODIMENTS

An embodiment of the present invention will be described below with reference to drawings.

### EMBODIMENT

### (Configuration)

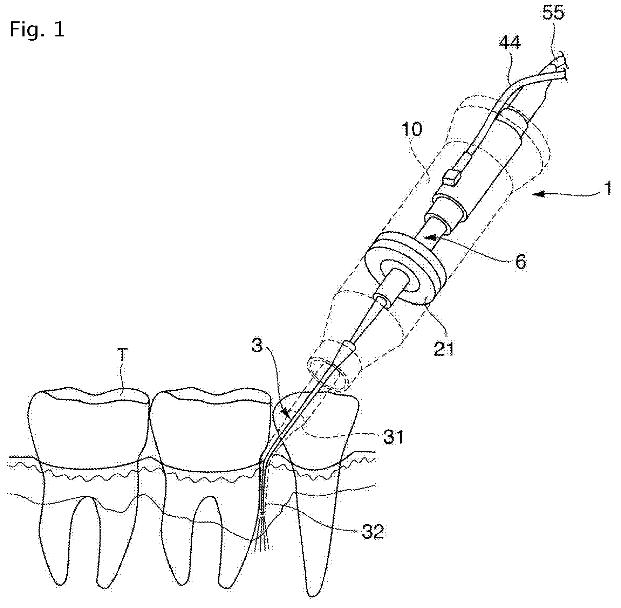

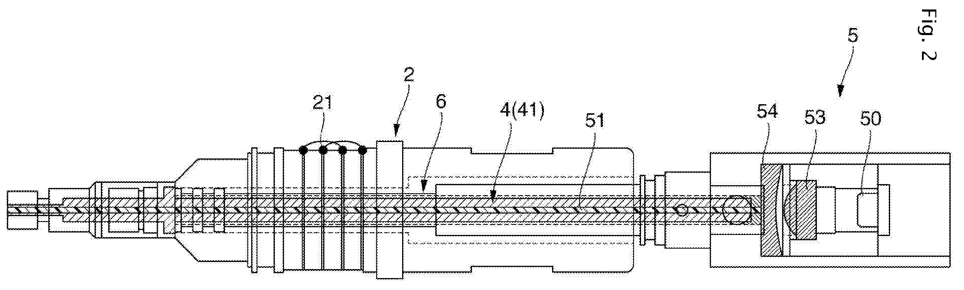

First, a configuration of a dental treatment device 1 of the present embodiment will be described with reference to Fig. 1. The dental treatment device 1 of the present embodiment includes a cover 10 covering a vibration unit 2, a flow channel 4, and an optical system 5 (see Fig. 2). A supply tube 44 configured to supply an antimicrobial agent described later and a cord 55 configured to supply electricity are connected to a base end of the cover 10. A chip unit 3 is detachably attached to a tip end of the cover 10. Using the chip unit 3, removal of dental calculus or plaque and disinfection/sterilization are performed.

The dental treatment device 1 is operated in the oral cavity of a patient with the dental treatment device 1 being held by the hand of an operator. Thus, in designing, size reduction, operability, and safety of the entire device are, as basic requirements, taken into consideration.

The dental treatment device 1 of the present embodiment includes, as illustrated in Fig. 2, the vibration unit 2 configured to vibrate by a vibrator 21 configured to generate ultrasonic vibrations, the chip unit 3 which is connected to the vibration unit 2 and to which ultrasonic vibrations are transmitted, the flow channel 4 configured to discharge the antimicrobial agent from the chip unit 3, and the optical system 5 configured to irradiate a laser beam for photo-decomposing the antimicrobial agent from the chip unit 3.

The vibration unit 2 is made of an alloy resistant to corrosion, such as stainless, and is formed in a substantially cylindrical shape. The vibrator 21 configured to generate ultrasonic vibrations is attached to a predetermined position of the vibration unit 2. The vibrator 21 is formed of a piezoelectric element combination, and is driven and vibrates by voltage supplied from a controller (not shown) and having a predetermined frequency. A vibration direction is the axial direction (the longitudinal direction) of the cover 10.

A shaft portion 6 assembled together with a light source 50 is inserted into the vibration unit 2 from a base end side thereof along the axial direction (the longitudinal direction) of the vibration unit 2. The outer diameter of the shaft portion 6 is smaller than the inner diameter of the vibration unit 2, and therefore, the shaft portion 6 does not directly contact the vibration unit 2.

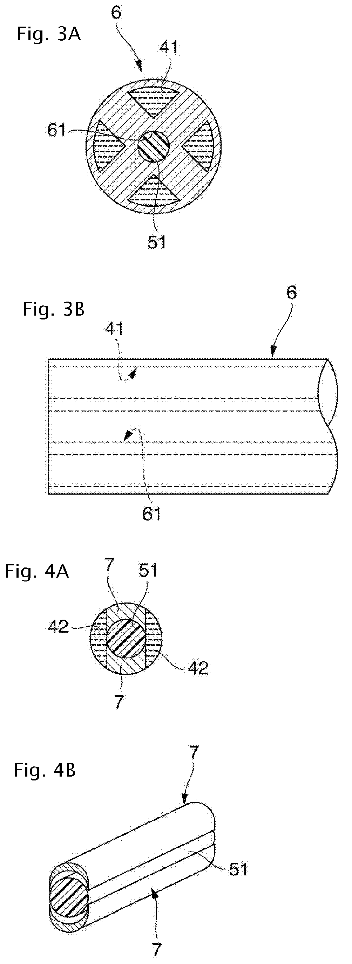

As illustrated in views of Figs. 3A and 3B, the shaft portion 6 is a member formed, as a whole, in a substantially circular columnar shape. The shaft portion 6 includes a hole 61 penetrating a shaft center, and flow channels 41 arranged about the hole 61.

The hole 61 is a circular through-hole penetrating the center of the shaft portion 6 in the axial direction thereof, and a fiber rod 51 forming the optical system 5 is inserted into the hole 61. The fiber rod 51 is a so-called "optical fiber," and has a triplex structure of a core, a clad, and a coating. The structure is employed, in which the refractive index of the core is higher than that of the clad and light propagates through the center core by total reflection and refraction.

The flow channels 41 are, as illustrated in Fig. 3A, formed as four fan-shaped holes, and are arranged respectively at the positions of nine o'clock, twelve o'clock, three o'clock, and six o'clock. Note that the shape and arrangement of the flow channels 41 are not limited to those of the present embodiment, and other shapes and arrangements may be employed. For example, the flow channels 41 may be circular holes, and more holes may be formed.

The chip unit 3 is made of, e.g., an alloy, and is formed in a hollow hook shape (an L-shape) with a pointed tip end. The chip unit 3 is connected to a tip end side of the vibration unit 2 to vibrate, and therefore, can remove dental calculus or plaque adhering to teeth T. Note that the shape of the chip unit 3 is not limited to the hook shape, and may be a linear shape or an arc shape.

A light guide 52 configured to transmit a laser beam from a base end 31 to a tip end 32 of the chip unit 3 is fitted into the chip unit 3 of the present embodiment. The light guide 52 is made of resin having a higher refractive index than that of the antimicrobial agent to be used. For example, acrylic resin can be used.

The light guide 52 is inserted and loosely fitted into the chip unit 3. Thus, a flow channel 43 through which the antimicrobial agent flows is formed between an inner surface of the chip unit 3 and an outer surface of the light guide 52.

The flow channel 4 includes an inlet port 40 connected to the supply tube 44, the flow channels 41 formed on a base end side of the shaft portion 6, flow channels 42 formed by spaces other than spacers 7 and the fiber rod 51 on the tip end side in the vibration unit 2, and the flow channel 43 in the chip unit 3.

The optical system 5 includes the light source 50 serving as a semiconductor light source configured to generate a laser beam, lenses 53, 54 configured to condense the generated laser beam, the fiber rod 51 configured to transmit the condensed laser beam in the shaft portion 6, and the light guide 52 configured to transmit the laser beam in the chip unit 3. That is, the optical system 5 of the present embodiment is separated from the flow channel 4 for the antimicrobial agent.

The light source 50 and the lenses 53, 54 are fixed to the base end side of the shaft portion 6 to have a predetermined relative position relationship. The light source 50 is, e.g., a semiconductor laser (LD) or a light emitting diode (LED).

The wavelength of the laser beam generated by the light source 50 is set within a range suitable for photo-decomposition of the antimicrobial agent. For example, in the case of selecting a hydrogen peroxide solution as the antimicrobial agent, the wavelength of the laser beam is preferably 405 nm.

### (Operations)

Next, operations of the dental treatment device 1 of the present embodiment will be described. According to the dental treatment device 1 of the present embodiment, cleaning and disinfection/sterilization described below can be performed simultaneously. In order to perform cleaning (e.g., removal of dental calculus), the controller operates to vibrate the vibrator 21 to generate ultrasonic vibrations. The ultrasonic vibrations are transmitted to the chip unit 3 via the vibration unit 2. Then, the tip end 32 of the chip unit 3 is pressed against, e.g., dental calculus to crush the dental calculus by the ultrasonic vibrations.

Meanwhile, in order to perform disinfection/sterilization, the controller operates to light up the light source 50. Light emitted from the light source 50 is condensed by the lenses 53, 54, and then, enters a base end of the fiber rod 51. The incident light is transmitted not in the antimicrobial agent but in the fiber rod 51, and then, reaches the light guide 52. Subsequently, the light transmitted in the light guide 52 is emitted from a tip end of the light guide 52. Such light generates hydroxyl radicals in the antiseptic solution, and the hydroxyl radicals perform disinfection (sterilization/antisepsis).

### (Advantageous Effects)

Next, a list of advantageous effects of the dental treatment device 1 of the present embodiment will be described.
(1) The dental treatment device 1 of the present embodiment is the dental treatment device 1 for removing dental calculus or plaque and disinfecting teeth. This dental treatment device 1 includes the vibration unit 2 configured to vibrate by the vibrator 21 configured to generate ultrasonic vibrations, the chip unit 3 which is connected to the vibration unit 2 and to which the ultrasonic vibrations are transmitted, the flow channel 4 configured to discharge the antimicrobial agent from the chip unit 3, and the optical system 5 configured to irradiate the laser beam for photo-decomposing the antimicrobial agent from the chip unit 3, the optical system 5 having the optical path separated from the flow channel 4.
   Thus, scattering of the laser beam by the antimicrobial agent is not liable to occur. Thus, a high-intensity laser beam is irradiated from a tip end of the chip unit 3, leading to efficient photo-decomposition of the antimicrobial agent.
(2) The light guide 52 configured to guide, as the optical system 5, the laser beam to the tip end of the chip unit 3 is disposed at the chip unit 3, and the fiber rod 51 extending from the vicinity of the light source 50 of the optical system 5 to a base end portion 52a of the light guide 52 is further provided.
   The light source 50 is disposed on a base end side of the dental treatment device 1, and the vibrator 21 is disposed on a tip end side of the dental treatment device 1. Thus, the size of the entirety of the dental treatment device 1 can be reduced, and attenuation of the laser beam can be reduced.
   That is, in order to efficiently vibrate the chip unit 3, the vibrator 21 is preferably positioned on the tip end side. Accordingly, in order to reduce the size of the entire device, the light source 50 is disposed on the base end side. Thus, attenuation of the laser beam is reduced in such a manner that the fiber rod 51 configured to transmit the laser beam from the light source 50 on the base end side to the tip end side is disposed.
(3) The shaft portion 6 assembled together with the light source 50 is inserted into the vibration unit 2 from the base end side thereof along the axial direction of the vibration unit 2. The hole 61 into which the fiber rod 51 is inserted is positioned at the center of the shaft portion 6, and the flow channel 41 is positioned about the hole 61.
   Thus, the shaft portion 6 can form the flow channels 41 while maintaining the relative position relationship among the components of the optical system 5. In other words, both of the flow channel 4 and the optical path can be ensured in a narrow space in the vibration unit 2.
(4) The shaft portion 6 is formed not to directly contact the inside of the vibration unit 2. This prevents transmission of ultrasonic vibrations of the vibration unit 2 to the shaft portion 6, resulting in prevention of failure of the light source 50 and the lenses 53, 54.
(5) The spacer 7 sandwiching the fiber rod 51 are arranged on the tip end side in the vibration unit 2. Thus, the optical path and the flow channel 4 can be separated from each other while maintaining the position of the fiber rod 51 even in a narrow space.
(6) The antimicrobial agent can be selected from hydrogen peroxide, an antimicrobial agent containing catechins, or a polyphenol solution. Thus, periodontal sterilization can be performed by hydrogen peroxide decomposition using the antimicrobial agent containing catechins. Alternatively, wound treatment in the oval cavity can be promoted using polyphenol, or oxidative stress (reactive oxygen species produced by inflammatory cells such as neutrophils) in inflammation can be relieved.
(7) When the hydrogen peroxide is used as the antimicrobial agent, the wavelength of the laser beam can be 405 nm. This can efficiently generate hydroxyl radicals. Further, light having such a wavelength is safe to the human body, and is effective for reduction in growth of periodontal disease-causing bacteria. Thus, a disinfection effect can be further enhanced.

The embodiment of the present invention has been described above in detail with reference to the drawings, but the specific configuration is not limited to the above-described embodiment. The present invention includes design change without departing from the gist of the present invention.

For example, the wavelength of the laser beam may be adjustable within a range of 400 to 500 nm. If the wavelength is changeable depending on a periodontal disease condition, a device with favorable cost performance can be provided.

Moreover, it has been described in the embodiment that the flow channel for antimicrobial agent and the optical path for laser beam are completely separated from each other. However, the present invention is not limited to this case. The scope of the technical idea of the present invention contains a region where the flow channel and the optical path overlap, without being separated from each other, with each other by an extremely short distance.

### REFERENCE SIGNS LIST

- 1: dental treatment device
- 2: vibration unit
- 21: vibrator
- 3: chip unit
- 4: flow channel
- 7: spacer
- 41: flow channel
- 5: optical system
- 50: light source
- 51: fiber rod
- 52: light guide
- 6: shaft portion
- 61: hole

## Claims

1. A dental treatment device for removing dental calculus or plaque and disinfecting teeth, comprising:
a vibration unit configured to vibrate by a vibrator configured to generate ultrasonic vibrations;
a chip unit which is connected to the vibration unit and to which the ultrasonic vibrations are transmitted;
a flow channel configured to discharge an antimicrobial agent from the chip unit; and
an optical system configured to irradiate a laser beam for photo-decomposing the antimicrobial agent from the chip unit, the optical system having an optical path separated from the flow channel.

2. The dental treatment device according to claim 1, further comprising:
a light guide disposed at the chip unit, and configured to guide, as the optical system, the laser beam to a tip end of the chip unit, and
a fiber rod extending from a vicinity of a light source of the optical system to a base end portion of the light guide.

3. The dental treatment device according to claim 2, wherein
a shaft portion assembled together with the light source is inserted into the vibration unit from a base end side thereof along an axial direction of the vibration unit, and
a hole into which the fiber rod is inserted is positioned at a center of the shaft portion, and the flow channel is positioned about the hole.

4. The dental treatment device according to any one of claims 1 to 3, wherein
the shaft portion is formed not to directly contact an inside of the vibration unit.

5. The dental treatment device according to any one of claims 2 to 4, wherein
spacer members sandwiching the fiber rod are arranged on a tip end side in the vibration unit.

6. The dental treatment device according to any one of claims 1 to 5, wherein
the antimicrobial agent is selected from hydrogen peroxide, an antimicrobial agent containing catechins, or a polyphenol solution.

7. The dental treatment device according to any one of claims 1 to 6, wherein
when the hydrogen peroxide is used as the antimicrobial agent, a wavelength of the laser beam is 405 nm.
